# EUROPÄISCHE PATENTSCHRIFT

(11) **EP 0 910 293 B1**
(45) Veröffentlichungstag und Bekanntmachung des Hinweises auf die Patenterteilung: **26.02.2003**
(21) Anmeldenummer: 97933629.4
(22) Anmeldetag: 11.07.1997
(51) Int. Cl.: A61B 17/28, A61B 17/11

(54) **INSTRUMENT MIT ZWEI VONEINANDER UNABHÄNGIGEN ZANGEN**
INSTRUMENT WITH INDEPENDENT PLIERS
INSTRUMENT AVEC DEUX PINCES INDEPENDANTES

(30) Priorität: 11.07.1996 DE 19627992
(43) Veröffentlichungstag der Anmeldung: 28.04.1999
(73) Patentinhaber: Karl Storz GmbH & Co. KG, 78532 Tuttlingen (DE)
(72) Erfinder: MANHES, Hubert, F-03200 Vichy (FR)
(74) Vertreter: Münich, Wilhelm, Dr.
(86) Internationale Anmeldenummer: DE9701468
(87) Internationale Veröffentlichungsnummer: WO98002103

(56) Entgegenhaltungen:
- EP-A- 0 592 243
- EP-A- 0 646 358
- WO-A-93/07816
- WO-A-94/18893
- DE-A- 3 909 999
- DE-C- 3 504 292
- DE-C- 4 324 254
- US-A- 5 395 367

## Beschreibung

### Technisches Gebiet

Die Erfindung bezieht sich Instrument zum Einsatz bei endoskopischen Eingriffen.

### Stand der Technik

Ein Instrument, von dem bei der Formulierung des Oberbegriffs des Patentanspruchs 1 ausgegangen wird, ist aus der DE 43 24 254 C1 bekannt.

Nun ist es bei einer Reihe von endoskopischen Eingriffen erforderlich, zwei Gewebeteile, die voneinander getrennt sind, "zusammenzuziehen", in diesem Zustand festzuhalten und anschließend beispielsweise durch einen Näh- oder Klebevorgang miteinander zu verbinden.

Beispiele für derartige Eingriffe sind die Operation von gerissenen Bändern in einem Gelenk, wie beispielsweise dem Kniegelenk, oder die in-vitro-Fertilisation der Eileiter. Ein weiteres Beispiel ist in dem Artikel von D.J. TIBBS et al. "Arterial Replacement with Minimal Interruption of Blood-Flow, erschienenen in "The Lancet, 1958, pp. 292 bis 294" beschrieben.

Die Durchführung dieser Eingriffe erfordert mit herkömmlichen Instrumenten nicht nur vergleichsweise viel Zeit, sondern auch ein großes manuelles Geschick des den Eingriff ausführenden Arztes, da dieser mehrere getrennt in den menschlichen Körper eingeführte Instrumente handhaben und ihre Bewegung koordinieren muß.

Auch das aus der DE 43 24 254 C1, der als nächstliegender Stand der Technik anzusehen ist, bekannte Instrument ist für die Durchführung derartiger Eingriffe nur bedingt geeignet, da die beiden unabhängig voneinander in einen Schaft mit mehreren Kanälen eingeführten abwinkelbaren Zangen keine koordinierte Bewegung im Sinne einer gezielten Annäherung der beiden Zangen aneinander gestatten. Zudem sind die einzelnen Kanäle an den Spitzen eines gleichseitigen Dreiecks angeordnet, so daß die Manipulation des zusammengezogenen Gewebeteils durch ein in den dritten Kanal eingeführtes Instrument erschwert wird. Weiterhin sind die Maulteils ungünstig zu der Schwenkachse orientiert, so daß ein "Zusammenziehen" von empfindlichen Gewebeteilen nicht möglich ist.

### Darstellung der Erfindung

Der Erfindung liegt die Aufgabe zugrunde, ein Instrument zum Einsatz bei endoskopischen Eingriffen anzugeben, durch das das "Zusammenziehen" zweier Gewebeteile, die voneinander getrennt sind, das Festhaltern der Gewebeteile im zusammengezogenen Zustand und das anschließende Manipulieren, also beispielsweise das Verbinden durch einen Näh- oder Klebevorgang vereinfacht wird.

Eine erfindungsgemäße Lösung dieser Aufgabe ist im Patentanspruch 1 angegeben. Weiterbildungen der Erfindung sind Gegenstand der Ansprüche 2 folgende.

Erfindungsgemäß sind beide Zangenelemente jeweils als Einheit um parallele Schwenkachsen derart schwenkbar, daß ihr gegenseitiger Abstand in Richtung der Instrumenten-Querachse geändert werden kann. Da diese Schwenkachsen zur Längsachse des Instruments wenigstens annähernd senkrecht sind, ist der Verstellbereich für den Abstand der Zangenelemente wesentlich größer als der Durchmesser des Instruments, der durch die zur Verfügung stehende maximale "Körperöffnung" beschränkt wird.

Zwischen den Mäulern der beiden Zangenelemente ist die distale Austrittsöffnung eines durchgehenden Kanals vorgesehen, der so ausgebildet ist, daß in ihn (u.a.) ein Operationsinstrument eingesetzt werden kann, das mit den Mäulern der beiden Zangenelemente zusammenwirkt.

Durch diese erfindungsgemäße Ausbildung ist bei der Verbindung von voneinander getrennten Gewebeteilen folgende Vorgehensweise möglich:

Das erfindungsgemäße Instrument wird in den Hohlraum eingeführt, in dem sich die zu verbindenden Gewebeteile befinden. Die freien Enden der Gewebeteile, also beispielsweise der Eileitertuben werden mit dem Maul jeweils eines Zangenelements des Instruments ergriffen. Anschließend werden die Zangenmäuler der Zangenelemente in einer Richtung senkrecht zur Längsachse des Instruments "aufeinander zu bewegt". Da die beiden Zangenelemente des Instruments die Gewebeteile festhalten, werden auch die freien Enden der zu verbindenden Gewebeteile aufeinander zu bewegt. Sobald sich die freien Enden in einer Position befinden, in der die Ausführung des Verbindungsvorgangs möglich ist, führt der behandelnde Arzt den Verbindungsvorgang aus. Während des Verbindungsvorgangs und gegebenenfalls auch noch danach werden die zu verbindenden Teile mit dem erfindungsgemäßen Instrument festgehalten. Selbstverständlich kann das erfindungsgemäße Instrument auch für andere Behandlungs- bzw. Bearbeitungsvorgänge im menschlichen oder tierischen Körper oder bei technischen Einsätzen verwendet werden.

Gemäß Anspruch 2 sind das oder die schwenkbaren Maulteile jedes Zangenelements um eine Achse schwenkbar, sind, die senkrecht auf der Schwenkachse des Zangenelements steht. Diese Orientierung, die der aus der DE 43 24 254 C1 bekannten Orientierung zwischen Zangenmaul und Schwenkachse entgegengesetzt ist, ermöglicht das Zusammenziehen auch empfindlicher Gewebeteile, da sich die Maulteile damit in etwa senkrecht zu der Richtung bewegen, in der die Gewebeteile durch Schwenken der Zangenelemente bewegt werden.

Gemäß Anspruch 3 ist vorgesehen, daß am proximalen Ende des Instruments für jedes Zangenelement ein Schwenk-Betätigungselement vorgesehen ist, durch dessen Betätigung der Schwenkwinkel und damit der Abstand zwischen den Zangenelementen in Richtung der Querachse geändert wird. Die Änderung des Schwenkwinkels hat dabei keinen Einfluß auf die Relativ-Stellung der Maulteile jedes Zangenelements zueinander. Damit ist es möglich, die zu verbindenden freien Gewebeteile präzise und ohne Schädigung des Gewebes zusammenzuführen.

Im Anspruch 4 ist eine Weiterbildung gekennzeichnet, gemäß der die Zangenelemente in Art bekannter Zangen aufgebaut sind, die insbesondere als Einheit in einen Schaft eingesetzt sind. Diese Ausbildung vereinfacht sowohl die Herstellung, die Lagerhaltung beim Hersteller als auch die Reinigung des erfindungsgemäßen Instruments. Dabei ist es ferner von Vorteil, wenn der Schaft in Art eines bekannten Trokars oder eines Laparoskops ausgebildet ist. Dieser Trokarschaft kann beispielsweise dann, wenn Eileiter-Tuben vernäht werden sollen, einen Außendurchmesser von 10 mm bis 13 mm haben (Ansprüche 19 bzw. 20).

Weiterhin ist es gemäß Anspruch 5 bevorzugt, wenn die Betätigungselemente für die Maulteile der Zangen Griffstücke - wie Scherengriffe, Zangengriffe oder dgl. - sind, die in die Stellung vorgespannt sind, in der das jeweilige Zangenmaul geschlossen ist. Diese Ausbildung hat den Vorteil, daß der Arzt beim Zusammenziehen der Gewebeteile die Maulteile der beiden Zangen nicht durch Festhalten der Griffstücke im geschlossenen Zustand halten muß.

Die Variation des Abstandes zwischen den beiden Zangen elementen kann prinzipiell auf die verschiedensten Arten erfolgen. Beispielsweise ist es möglich, wenigstens ein Zangenelement an einer elastischen und nach außen gebogenen Halterung zu halten. Mittels einer Linearführung oder einer Hülse wird dann das Zangenelement "nach innen gedrückt" bzw. der Anlenkpunkt verschoben.

Einen besonders großen Verstellbereich erhält man - gerade im Hinblick auf den begrenzten Durchmesser von Endoskopen - durch die in den Ansprüchen 6 folgende angegebenen Merkmale, die die Verschwenkung des distalen Endes der Zangenelemente bzw. Zangen als Einheit angeben:

Gemäß Anspruch 6 sind die Zangenmäuler Bestandteil an sich bekannter flexibler Zangen, deren distales Ende in ebenfalls an sich bekannter Weise abwinkelbar ist.

Die Schwenk- bzw. Abwinkelbewegung der Zangenmäuler kann auf die verschiedensten Arten hervorgerufen werden:

So können gemäß Anspruch 7 Übertragungselemente, wie beispielsweise Seilzüge, Schub- bzw. Zugstangen vorgesehen sein, die die Bewegung der Betätigungselemente auf die Zangenmäuler übertragen. Weiterhin ist es möglich, daß distal angeordnete Actuatoren vorgesehen sind, die den Schwenk- bzw. Abwinkelbewegung erzeugen (Anspruch 8). Die Actuatoren können gemäß Anspruch 9 insbesondere elektrisch betätigte Mikrostellemente, wie Mikromotoren sein.

Weiterhin ist es möglich, als Zangen starre Zangen zu verwenden, deren distales Ende als Einheit abgewinkelt werden kann, und deren Maulteile über eine Stange oder dgl. mit einem proximal angeordneten Betätigungselement verbunden sind (Anspruch 10). Insbesondere können Zangen verwendet werden, wie sie in der älteren DE-Patentanmeldung !96 25 241.5 der Karl Storz GmbH & Co., DE beschrieben sind.

Dabei ist es gemäß Anspruch 11 bevorzugt, wenn ein Ausgleichsmechanismus vorgesehen ist, der eine Änderung der relativen Winkelstellung der beiden Maulteile des Mauls beim Abwinkeln bzw. Schwenken des distalen Endes als Einheit verhindert, da dann beim Ändern des Abstandes der beiden Zangen das Gewebe weder geschädigt werden kann noch aus dem jeweiligen Maulteil herausgleiten kann.

Bei Verwendung einer starren Zange ist es weiterhin bevorzugt, wenn die Stange in an sich bekannter Weise im Bereich der Abwinkelung flexibel ausgebildet ist (Anspruch 12) und auf Zug oder Druck das Maul schließt (Anspruch 13).

Beim Einsatz einer "starren Zange" ist es weiterhin bevorzugt, wenn gemäß Anspruch 14 ein Stellelement und insbesondere eine Stellschraube (Anspruch 15) vorgesehen ist, durch dessen Betätigung das distale Ende abwinkelbar bzw. schwenkbar ist.

Die Stellschraube kann unter einem Winkel von 90° zur Längsachse des Instruments oder konzentrisch zur Längsachse des Instruments angeordnet sein (Ansprüche 16 oder 17).

Die Handhabung des erfindungsgemäßen Instruments wird weiter vereinfacht, wenn bestimmte Winkelstellungen der beiden Zangen indexiert sind, oder wenn die Stellschraube eine Rastung bei bestimmten Winkelstellungen aufweist (Anspruch 18).

In den zusätzlichen Kanal können die verschiedensten Instrumente eingesetzt werden:

Beispielsweise ist es gemäß Anspruch 21 möglich, eine Nähvorrichtung oder eine Klebevorrichtung einzusetzen.

Insbesondere dann, wenn distale und proximale Eileiter stümpfe nach Einführung eines Katheders miteinander verbunden werden sollen, ist es von Vorteil, wenn die Klebevorrichtung ein Katheder ist, der das Aufbringen eines Fibrinklebers gestattet (Anspruch 22).

Die Zangenmäuler des erfindungsgemäßen Instruments können prinzipiell in jeder bekannten Ausführungsform ausgebildet sein. Besonders vorteilhaft ist es jedoch, wenn nach Anspruch 23 die Zangenmäuler durch jeweils zwei Greifbacken gebildet werden. Diese Greifbacken können mit Zähnen versehen sein (Anspruch 24), so daß die jeweiligen Gewebeteile sicher gegriffen werden können.

Dabei kann nach Anspruch 25 die Form der Greifbacken den zusammenzufügenden Gewebeteilen angepaßt sein, so daß ein großflächiger Formschluß erreicht wird.

Dabei kann nach Anspruch 25 die Form der Greifbacken den zusammenzufügenden Gewebeteilen angepaßt sein, so daß ein großflächiger Formschluß erreicht wird.

Die Anpassung an unterschiedliche Operationsbedingungen wird dadurch erleichtert, daß die Zangenmäuler zusätzlich in Richtung der Instrumenten-Längsachse gegenüber dem Instrumentenkörper verschiebbar sind (Anspruch 26). Die Verschiebung jedes Zangenmauls kann gemäß Anspruch 27 dabei unabhängig von dem anderen Zangenmaul erfolgen. Durch die Verschiebbarkeit in Richtung der Längsachse kann u.a. der Winkelversatz ausgeglichen werden, der sich durch die Verschwenkung ergibt. Gegebenenfalls ist auch eine Zwangsführung zum Ausgleich des Winkelversatzes möglich.

### Kurze Beschreibung der Zeichnung

Die Erfindung wird nachstehend ohne Beschränkung des allgemeinen Erfindungsgedankens anhand von Ausführungsbeispielen unter Bezugnahme auf die Zeichnung exemplarisch beschrieben, auf die im übrigen bezüglich der Offenbarung aller im Text nicht näher erläuterten erfindungsgemäßen Einzelheiten ausdrücklich verwiesen wird. Es zeigen:

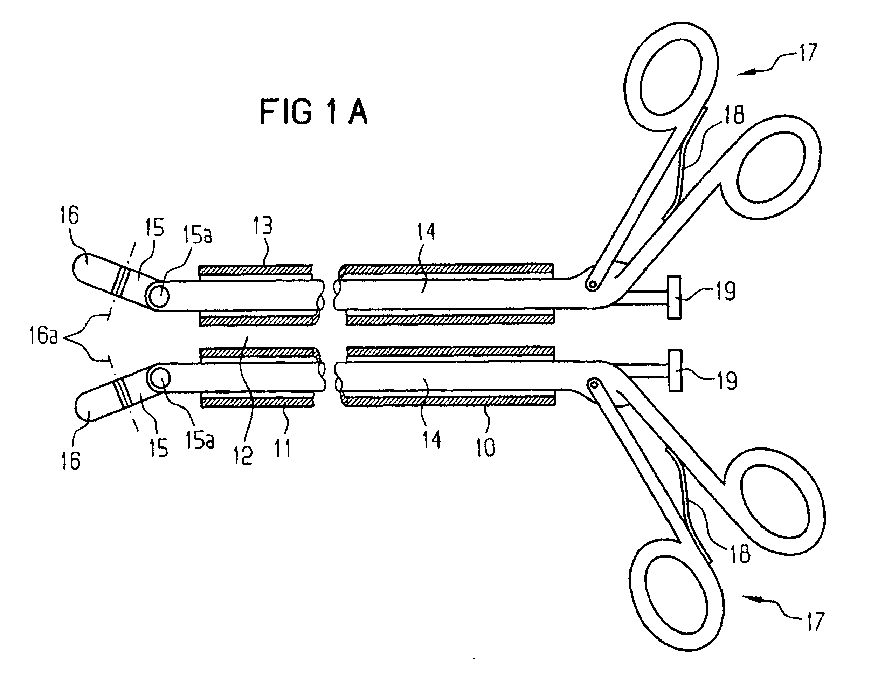

- Fig. 1a: eine teilweise geschnittene Seitenansicht eines ersten Ausführungsbeispiels eines erfindungsgemäßen Instruments gemäß Patentanspruch 1,

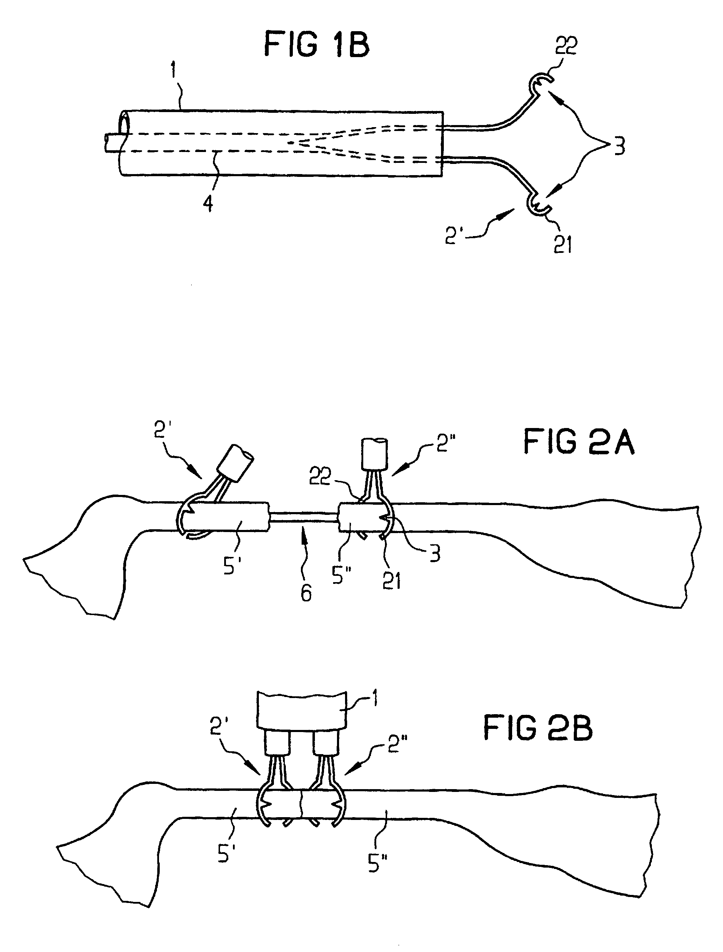

- Fig. 1b: eine schematische Darstellung eines nicht zur Erfindung gehörenden zweiten Ausführungsbeispiels, und

### Darstellung von Ausführungsbeispielen

Fig. 1a zeigt ein erstes Ausführungsbeispiel der Erfindung. Dieses Instrument weist einen Schaft 10 auf, in dem drei nebeneinanderliegende Kanäle 11 bis 13 vorgesehen sind. In die beiden äußeren Kanäle ist jeweils eine Zange 14 eingesetzt, deren distales Ende 15 als Einheit um eine Schwenkachse 15a schwenkbar ist.

Das distale Ende 15 umfaßt ferner Maulteile 16 der beiden Zangen; die Maulteile 16 sind um eine Achse 16a schwenkbar, die senkrecht auf der Achse 15a steht (in der Draufsicht in Figur 1a ist lediglich eines der beiden Maulteile zu sehen.

Zur Betätigung der Maulteile sind in an sich bekannter Weise Scherengriffe 17 vorgesehen, die durch eine Feder 18 in die Stellung vorgespannt sind, in der die Maulteile 16 geschlossen sind. Zur Abwinkelung des distalen Endes 15 als Einheit ist jeweils ein Betätigungselement 19 vorgesehen, das koaxial zur Zangen-Längsachse angeordnet ist. Die Betätigungselemente 17 und 19 sind über Stangen oder Seilzüge mit den jeweiligen distalen Elementen verbunden.

Bevorzugt weist das Betätigungselement 19 eine Indexierung oder Rastung auf, so daß bestimmte Abwinkelungen der Elemente 15 leicht eingestellt werden können.

Zusätzlich sind die Zangen 14 in ihren jeweiligen Kanälen in Richtung ihrer Längsachse verschiebbar, so daß umfangreiche Manipulationen möglich sind.

In den mittleren Kanal 12 kann ein Instrument, wie beispielsweise ein Katheder zum Aufbringen eines Klebers, eine Nähvorrichtung oder auch eine weitere Zange eingesetzt werden.

Die Funktionsweise des erfindungsgemäßen Instruments wird nachstehend nach der Beschreibung des zweiten Ausführungsbeispiels in Verbindung mit Figur 1b erläutert.

Fig. 1b zeigt lediglich das distale Ende eines zweiten Ausführungsbeispiels eines erfindungsgemäß ausgebildeten Instruments zum Einsatz bei endoskopischen Eingriffen. An dem distalen Ende eines nur teilweise dargestellten Instrumentenkörpers 1 sind zwei unabhängig voneinander durch nicht gezeigte Betätigungselemente betätigbare Zangenmäuler 2' bzw. 2" (Fig. 2) vorgesehen, von denen in der Seitenansicht der Fig. 1b nur eines (2') mit den Greifbacken 21 und 22 dargestellt ist.

Die Form der mit Zähnen 3 versehenen Greifbacken 21 und 22 ist den zusammenzufügenden Gewebeteilen, im dargestellten Fall Eileitertuben angepaßt.

Erfindungsgemäß kann der Abstand der Zangenmäuler 2' und 2" in Richtung der Instrumenten-Querachse durch eine Schwenk- bzw. Abwinkelbewegung jedes Zangenmauls geändert werden. Dies zeigen die Fig. 2a und 2b.

Bei dem in den Fig. 1b und 2 dargestellten ersten Ausführungsbeispiel sind die beiden Zangenmäuler an Haltern befestigt, die elastisch in Richtung der Querachse nach außen vorgespannt sind, so daß sie in ihrer Ruhestellung den maximal möglichen Abstand haben. Zur Verringerung des Abstandes wird mittels eines proximal angeordneten weiteren - ebenfalls nicht dargestellten - Betätigungselement beispielsweise eine Hülse verschoben, so daß die Zangenmäuler "nach innen" gedrückt werden.

In dem Instrumentenkörper 1 sind Übertragungselemente 4 vorgesehen sind, die die Schwenk- bzw. Abwinkelbewegung sowie die Öffnungs- und Schließbwegung von den nicht dargestellten Betätigungselementen zu den Zangenmäulern 2' bzw. 2" übertragen.

Die Funktionsweise des in Fig. 1 dargestellten Instruments soll im folgenden unter Bezugnahme auf die Fig. 2a und 2b erläutert werden, in denen gleiche Teile wie in Fig. 1b mit den selben Bezugszeichen versehen sind:

Die zusammenzufügenden Tubenstümpfe 5' bzw. 5" des Uterus 5 werden, nachdem gegebenenfalls ein Katheder 6 eingeführt worden ist, mit den Greifbacken der Zangen mäuler 2' und 2" gegriffen (Fig. 2a). Nach Beendigung des Greifvorgangs werden durch eine Bewegung der Zangenmäuler 2' bzw. 2" relativ zum Instrumentenkörper 1 - gegebenenfalls verbunden mit einer Linearverschiebung eines oder beider Zangenmäuler - die Stumpfenden zusammengeführt, so daß sie sich berühren. Anschließend können die Stumpfenden der Tubenstümpfe 5' und 5" verklebt oder vernäht werden.

Hierzu ist das Instrument mit einem Kanal versehen, der den Einsatz des Klebers entweder direkt oder indirekt über einen Führungskatheder gestattet.

## Patentansprüche

1. Instrument zum Einsatz bei endoskopischen Eingriffen Mit einem Schaft (10), der an seinem distalen Ende zwei unabhängig voneinander durch Betätigungselemente (17) betätigbare Zangenelemente (15) aufweist, wobei die Zangenelemente (15) jeweils zwei um eine gemeinsame Achse (16a) schwenkbare Maulteile (16) aufweisen, und die Zangenelemente (15) um jeweils eine Schwenkachse (15a) schwenkbar sind, wobei die Schwenkachsen (15a) parallel zueinander und senkrecht zur Längsachse des Instruments angeordnet sind, so daß der zur Längsachse des Instruments senkrechte Abstand der Zangenelemente (15) voneinander geändert werden kann,
**dadurch gekennzeichnet, daß** die Achsen (16a), um welche die Maulteile (16) schwenkbar sind, senkrecht zu den Schwenkachsen (15a) angeordnet sind, um welche die Zangenelemente (15) schwenkbar sind, und der Schaft (10) einen durchgehenden Kanal (12) zum Einsetzen eines Operationsinstruments aufweist, dessen distale Austrittsöffnung zwischen den Zangenelementen (15) angeordnet ist.

2. Instrument nach Anspruch 1,
**dadurch gekennzeichnet, dass** am proximalen Ende des Instruments für jede Zange (14) ein Schwenk-Betätigungselement(19) vorgesehen ist, dessen Betätigung die Schwenkstellung der jeweiligen Zange (14) ändert, ohne daß die Relativ-Stellung der beiden Maulteile(16) dieser Zange(14) zueinander geändert wird.

3. Instrument nach einem der Ansprüche 1 bis 2 ,
**dadurch gekennzeichnet, dass** die Zangen (14) in Art bekannter Zangen aufgebaut sind, die insbesondere als Einheit in einen Schaft(10) eingesetzt sind.

4. Instrument nach einem der Ansprüche 1 bis 3,
**dadurch gekennzeichnet, dass** die Betätigungselemente(19) für die Maulteile(16) der Zangen (14) Griffstücke sind, die in die Stellung vorgespannt sind, in der das Zangenmaul geschlossen ist.

5. Instrument nach einem der Ansprüche 1 bis 4,
**dadurch gekennzeichnet, dass** die Zangen(14) in Art flexibler Zangen aufgebaut sind, deren distales Ende(15) in an sich bekannter Weise abwinkelbar ist.

6. Instrument nach einem der Ansprüche 1 bis 5,
**dadurch gekennzeichnet, dass** Übertragungselemente(4) vorgesehen sind, die die Schwenk- bzw. Abwinkelbewegung erzeugen.

7. Instrument nach einem der Ansprüche 1 bis 6,
**dadurch gekennzeichnet, dass** distal angeordnete Actuatoren vorgesehen sind, die den Schwenk- bzw. Abwinkelbewegung der Zangenmäuler (2', 2") erzeugen.

8. Instrument nach Anspruch 7,
**dadurch gekennzeichnet, dass** die Actuatoren elektrisch betätigte Mikrostellemente sind.

9. Instrument nach einem der Ansprüche 1 bis 4,
**dadurch gekennzeichnet, dass** die Zangen(14) starre Zangen sind, deren distales Ende(15) um eine Achse abwinkelbar oder durch Verschieben einer Hülse oder dgl. gegen eine elastische Vorspannung verschiebbar ist, und deren Maulteile(16) über eine Stange oder dgl. mit einem proximal angeordneten Betätigungselement(19) verbunden sind.

10. Instrument nach Anspruch 9,
**dadurch gekennzeichnet, dass** ein Ausgleichsmechanismus vorgesehen ist, der eine Änderung der relativen Winkelstellung der beiden Maulteile(16) des Mauls beim Abwinkeln bzw. Schwenken des distalen Endes(15) als Einheit verhindert.

11. Instrument nach Anspruch 9 oder 10,
**dadurch gekennzeichnet, dass** die Stange in an sich bekannter Weise im Bereich der Abwinkelung flexibel ausgebildet ist.

12. Instrument nach einem der Ansprüche 9 bis 11,
**dadurch gekennzeichnet, dass** die Stange auf Zug oder Druck das Maul schließt.

13. Instrument nach einem der Ansprüche 9 bis 12,
**dadurch gekennzeichnet, dass** ein Stellelement vorgesehen ist, durch dessen Betätigung das distale Ende(15) abwinkelbar bzw. schwenkbar ist.

14. Instrument nach Anspruch 13,
**dadurch gekennzeichnet, dass** das Stellelement eine Stellschraube ist.

15. Instrument nach Anspruch 14,
**dadurch gekennzeichnet, dass** die Stellschraube unter einem Winkel von 90° zur Längsachse des Instruments angeordnet ist.

16. Instrument nach Anspruch 14,
**dadurch gekennzeichnet, dass** die Stellschraube konzentrisch zur Längsachse des Instruments angeordnet ist.

17. Instrument nach einem der Ansprüche 1 bis 16,
**dadurch gekennzeichnet, dass** bestimmte Winkelstellungen der beiden Zangen(14) indexiert sind, oder dass die Stellschraube eine Rastung bei bestimmten Winkelstellungen aufweist.

18. Instrument nach einem der Ansprüche 1 bis 17,
**dadurch gekennzeichnet, dass** das Instrument in einen Trokarschaft einsetzbar ist.

19. Instrument nach Anspruch 18,
**dadurch gekennzeichnet, dass** der Trokarschaft einen Außendurchmesser von 10 bis 13 mm hat.

20. Instrument nach einem der Ansprüche 1 bis 19,
**dadurch gekennzeichnet, dass** in den durchgehenden Kanal(12) eine Nähvorrichtung oder eine Klebevorrichtung einsetzbar ist.

21. Instrument nach Anspruch 20,
**dadurch gekennzeichnet, dass** die Klebevorrichtung ein Katheder ist, der das Aufbringen eines Fibrinklebers gestattet.

22. Instrument nach Anspruch 7 oder 8,
**dadurch gekennzeichnet. daß** die Zangenmäuler (2', 2") durch jeweils zwei Greif- oder Scherenbacken gebildet werden.

23. Instrument nach Anspruch 22,
**dadurch gekennzeichnet, daß** die Greifbacken (2', 2") mit Zähnen (3) versehen sind.

24. Instrument nach Anspruch 22 oder 23,
**dadurch gekennzeichnet, daß** die Form der Greifbacken (2', 2") den zusammenzufügenden Gewebeteilen angepaßt ist.

25. Instrument nach einem der Ansprüche 1 bis 24,
**dadurch gekennzeichnet, daß** die Zangenmäuler (2', 2") zusätzlich in Richtung der Instrumenten-Längsachse verschiebbar sind.

26. Instrument nach Anspruch 25,
**dadurch gekennzeichnet, daß** jedes Zangenmaul (2') unabhängig von dem anderen Zangenmaul (2") verschiebbar ist.

## Claims

1. Instrument for use in endoscopic surgery, comprising a shaft (10) having at the distal end thereof two forceps elements (15) adapted to be independently actuated by actuators (17), the forceps elements (15) each having two jaw members (16) adapted to be swivelled around a mutual axis (16a), the forceps elements (15) each being adapted to be swivelled around a pivot axis (15a), and the pivot axes (15a) being disposed to be parallel to each other and perpendicular to the longitudinal axis of the instrument, so that the spacing of the forceps elements (15) from each other perpendicular to the longitudinal axis of the instrument can be changed,
**characterized in that** the axes (16a) around which the jaw members (16) are adapted to be swivelled are disposed to be perpendicular to the pivot axes (15a) around which the forceps elements (15) are adapted to be swivelled, and that the shaft (10) has a through passage (12) for the insertion of a surgical instrument, with a distal exit opening disposed between the forceps elements (15).

2. Instrument according to claim 1,
**characterized in that** a swivel-actuator (19) for each forceps (14) is provided at the proximal end of the instrument, the actuation of which changes the swivel position of the respective forceps (14) without changing the position of the two jaw members (16) of these forceps (14) relative to each other.

3. Instrument according to any one of claims 1 to 2,
**characterized in that** the forceps (14) are designed in the manner of known forceps which, in particular, are inserted as a unit into a shaft (10).

4. Instrument according to any one of claims 1 to 3,
**characterized in that** the actuators (19) for the jaw members (16) of the forceps (14) are handle pieces that are biased into the position in which the forceps jaw is closed.

5. Instrument according to any one of claims 1 to 4,
**characterized in that** the forceps (14) are designed in the manner of flexible forceps having a distal end (15) that is bendable in a manner known per se.

6. Instrument according to any one of claims 1 to 5,
**characterized in that** transmission elements (4) are provided for producing the swivel or bending movement.

7. Instrument according to any of claims 1 to 6,
**characterized in that** distally disposed actuators are provided for producing the swivel or bending movement of the forceps jaws (2', 2").

8. Instrument according to claim 7,
**characterized in that** the actuators are electrically operated micro-control elements.

9. Instrument according to any one of claims 1 to 4,
**characterized in that** the forceps (14) are rigid forceps having a distal end (15) bendable about an axis, or displaceable by the sliding of a sleeve or the like against a resilient biasing force, and having jaw members (16) connected via a rod or the like to a proximally disposed actuator (19).

10. Instrument according to claim 9,
**characterized in that** an equalising mechanism is provided for preventing a change of the relative angular position of the two jaw members (16) of the jaw when the distal end (15) is bent or swivelled as a unit.

11. Instrument according to claim 9 or 10,
**characterized in that** the bending region of the rod is designed in a manner known per se.

12. Instrument according to any one of claims 9 to 11,
**characterized in that** the rod closes the jaw in response to being pulled or pushed.

13. Instrument according to any one of claims 9 to 12,
**characterized in that** a setting means is provided, the operation of which bends or swivels the distal end (15).

14. Instrument according to claim 13,
**characterized in that** the setting means is a setting screw.

15. Instrument according to claim 14,
**characterized in that** the setting screw is disposed at an angle of 90° to the longitudinal axis of the instrument.

16. Instrument according to claim 14,
**characterized in that** the setting screw is disposed to be concentric with the longitudinal axis of the instrument.

17. Instrument according to any one of claims 1 to 16,
**characterized in that** particular angular positions of the two forceps (14) are indexed, or that the setting screw has a catch at particular angular positions.

18. Instrument according to any one of claims 1 to 17,
**characterized in that** the instrument is adapted to be inserted into a trocar shaft.

19. Instrument according to claim 18,
**characterized in that** the trocar shaft has an outside diameter of 10 to 13 mm.

20. Instrument according to any one of claims 1 to 19,
**characterized in that** a suturing device or a device for effecting adhesion can be inserted into the continuous passage.

21. Instrument according to claim 20,
**characterized in that** said device for effecting adhesion is a catheter permitting the application of a fibrin adhesive.

22. Instrument according to claim 7 or 8,
**characterized in that** the forceps jaws (2', 2") are each formed by two clamping or scissors jaws.

23. Instrument according to claim 22,
**characterized in that** the clamping jaws (2', 2") are provided with teeth (3).

24. Instrument according to claim 22 or 23,
**characterized in that** the shape of the clamping jaws (2', 2") is conformed to the tissue portions to be joined.

25. Instrument according to any one of claims 1 to 24,
**characterized in that** the forceps jaws (2', 2") are also slidable along the direction of the longitudinal axis of the instrument.

26. Instrument according to claim 25,
**characterized in that** each forceps jaw (2') is slidable independently from the other forceps jaw (2").

## Revendications

1. Instrument destiné à être utilisé lors d'interventions endoscopiques, avec une chemise (10) qui présente à son extrémité distale deux éléments de pince (15) aptes à être commandés indépendamment l'un de l'autre par des éléments d'actionnement (17), lesdits éléments de pince (15) présentant chacun deux mors (16) articulés sur un axe commun (16a), et les éléments de pince (15) étant chacun articulés autour d'un axe de pivotement (15a), les axes de pivotement (15a) étant agencés parallèlement entre eux et perpendiculairement à l'axe longitudinal de l'instrument, de sorte que la distance entre les éléments de pince (15) perpendiculairement à l'axe longitudinale de l'instrument peut être modifiée, **caractérisé en ce que** les axes (16a) sur lesquels sont articulés les mors (16) sont agencés perpendiculairement aux axes de pivotement (15a) sur lesquels sont articulés les éléments de pince (15), et **en ce que** la chemise (10) présente un canal traversant (12) destiné à recevoir un instrument chirurgical et dont l'ouverture de sortie distale est située entre les éléments de pince (15).

2. Instrument selon la revendication 1, **caractérisé en ce que** pour chaque pince (14) est prévu à l'extrémité proximale de l'instrument un élément d'entraînement en rotation (19), dont l'actionnement modifie la position angulaire des pinces (14) sans que la position relative des deux mors (16) de chaque pince (14) s'en trouve modifiée.

3. Instrument selon l'une des revendications 1 à 2, **caractérisé en ce que** les pinces (14) sont conçues à la manière de pinces connues insérées en particulier en bloc dans une chemise (10).

4. Instrument selon l'une des revendications 1 à 3, **caractérisé en ce que** les éléments d'actionnement (19) des mors (16) des pinces (14) sont des poignées qui sont rappelées dans la position dans laquelle la mâchoire de la pince est fermée.

5. Instrument selon l'une des revendications 1 à 4, **caractérisé en ce que** les pinces (14) sont conçues sous la forme de pinces flexibles dont on peut faire pivoter les extrémités distales (15) d'une manière connue en soi.

6. Instrument selon l'une des revendications 1 à 5, **caractérisé en ce qu'**il comprend des éléments de transmission (4) qui produisent le mouvement de pivotement ou de coudage.

7. Instrument selon l'une des revendications 1 à 6, **caractérisé en ce qu'**il comprend des actionneurs agencés distalement qui produisent le mouvement de pivotement ou de coudage des mâchoires de pince (2', 2'').

8. Instrument selon la revendication 7, **caractérisé en ce que** les actionneurs sont des microdispositifs de positionnement à commande électrique.

9. Instrument selon l'une des revendications 1 à 4, **caractérisé en ce que** les pinces (14) sont des pinces rigides dont l'extrémité distale (15) peut pivoter autour d'un axe ou être déplacée en translation par translation d'une douille ou similaire à l'encontre d'une force de rappel élastique et dont les mors (16) sont reliés à un élément d'actionnement (19) proximal par une tringle ou similaire.

10. Instrument selon la revendication 9, **caractérisé en ce qu'**il est prévu un mécanisme de compensation qui empêche toute modification de la position angulaire relative des deux mors (16) de la mâchoire lors du coudage ou du pivotement de l'extrémité distale (15) en tant qu'unité monobloc.

11. Instrument selon la revendication 9 ou 10, **caractérisé en ce que** la tringle est, d'une manière connue en soi, réalisée flexible au niveau du coude.

12. Instrument selon l'une des revendications 9 à 11, **caractérisé en ce que** la tringle ferme la mâchoire lorsqu'elle est sollicitée en traction ou en pression.

13. Instrument selon l'une des revendications 9 à 12, **caractérisé en ce qu'**il est prévu un élément de positionnement dont l'actionnement a pour effet de couder ou de faire pivoter l'extrémité distale (15).

14. Instrument selon la revendication 13, **caractérisé en ce que** l'élément de positionnement est une vis de réglage.

15. Instrument selon la revendication 14, **caractérisé en ce que** la vis de réglage est agencée à angle droit par rapport à l'axe longitudinal de l'instrument.

16. Instrument selon la revendication 14, **caractérisé en ce que** la vis de réglage est agencée concentriquement à l'axe longitudinal de l'instrument.

17. Instrument selon l'une des revendications 1 à 16, **caractérisé** en de que certaines positions angulaires des deux pinces (14) sont indexées ou en ce que la vis de réglage présente des crans correspondant à certaines positions angulaires.

18. Instrument selon l'une des revendications 1 à 17, **caractérisé en ce que** l'instrument peut être inséré dans une canule de trocart.

19. Instrument selon la revendication 18, **caractérisé en ce que** la canule de trocart présente un diamètre extérieur compris entre 10 et 13 mm.

20. Instrument selon l'une des revendications 1 à 19, **caractérisé en ce que** le canal traversant (12) peut recevoir un dispositif de suture ou un dispositif de collage.

21. Instrument selon la revendication 20, **caractérisé en ce que** le dispositif de collage est un cathéter, qui permet d'appliquer une colle de fibrine.

22. Instrument selon la revendication 7 ou 8, **caractérisé en ce que** les mâchoires de pince (2', 2'') sont chacune formées par deux mors de préhension ou deux branches de ciseaux.

23. Instrument selon la revendication 22, **caractérisé en ce que** les mors de préhension (2, 2") sont dotés de dents (3).

24. Instrument selon la revendication 22 ou 23, **caractérisé en ce que** la forme des mors de préhension (2', 2'') est adaptée aux parties de tissu devant être rapprochées bord à bord.

25. Instrument selon l'une des revendications 22 à 24, **caractérisé en ce que** les mâchoires de pince (2', 2'') sont, à titre complémentaire, mobiles en translation dans la direction de l'axe longitudinal de l'instrument.

26. Instrument selon la revendication 25, **caractérisé en ce que** chaque mâchoire de pince (2') est mobile en translation indépendamment de l'autre mâchoire de pince (2'').
